# EUROPEAN PATENT APPLICATION

(11) **EP 4 085 830 A1**
(43) Date of publication of application: **09.11.2022**
(21) Application number: 21171743.4
(22) Date of filing: 03.05.2021
(51) Int. Cl.: A61B 5/055, G01R 33/48, G06T 7/00, A61B 5/00

(54) **METHOD FOR DETECTING NERVE FIBERS IN A BRAIN OF A PATIENT, METHOD FOR DIAGNOSING OR PROGNOSING A DISEASE IN A PATIENT AND METHOD OF DETERMINING THE COURSE OF A DISEASE IN A PATIENT**

(71) Applicant: Otto-von-Guericke-University Magdeburg, 39104 Magdeburg (DE)
(72) Inventor: Liebe, Thomas, 39112 Magdeburg (DE); Kaufmann, Jörn, 39116 Magdeburg (DE)

(57) **Abstract**

In at least one embodiment, the method for detecting nerve fibers (NF) in a brain (B) of a patient comprises:
- receiving position information indicative for the position of a certain brain region (BR) of the brain,
- tracking nerve fibers starting from the position of the certain brain region in order to determine the nerve fibers connecting the certain brain region with other brain regions by using the position information and structural brain data, wherein
- the structural brain data are indicative for the distribution of nerve fibers in the brain.

## Description

A method for detecting nerve fibers in a brain of a patient is specified. Furthermore, a device, a computer program, a computer-readable data carrier, a method for diagnosing or prognosing a disease in or a psychological characteristic of a patient and a method of determining the course of a disease in or the psychological characteristic of a patient are specified.

### BACKGROUND OF THE INVENTION

The distribution, structure and/or amount of nerve fibers in the brain may be closely associated with psychological disturbances in neurodegenerative diseases. Therefore, knowing, e.g., the amount of nerve fibers connecting one brain region with another, may be helpful in diagnosing, prognosing or treating a disease.

It should be noted that these statements should not be construed as being admitted prior art. They are only made to illustrate the background of the presently disclosed concepts and may not have been made available to the public yet.

### DETAILED DESCRIPTION OF THE INVENTION

One object to be achieved is to provide an improved method for detecting nerve fibers in a brain of a patient. Further objects to be achieved are to provide a device configured to perform such a method, a computer program for carrying out such a method, a computer-readable data carrier for carrying out such a method, an improved method for diagnosing or prognosing a disease or psychological characteristics in a patient and an improved method of determining the course of a disease or psychological characteristics in a patient.

These objects are achieved, inter alia, by the subject matter of the independent claims. Advantageous embodiments and further developments are subject to the dependent claims and are also presented in the following description and in the figures.

Firstly, the method for detecting nerve fibers in a brain of a patient is specified.

According to at least one embodiment, the method comprises a step, in which position information are received. The position information may be indicative for the position of a certain brain region of the brain of the patient. Coordinates, e.g., Cartesian coordinates, of the certain brain region, e.g. of the center of the certain brain region, may be extractable form the position information. The position information may be received by a computer, e.g. via an interface such as a user interface, and/or may be received by a processor of a computer, e.g. from a storage medium. The position information may be computer data. The certain brain region may be a brain region of interest, e.g. in view of psychological diseases.

According to at least one embodiment, the method comprises a step of tracking nerve fibers starting from the position of the certain brain region in order to determine the nerve fibers connecting the certain brain region with other brain regions. For this purpose, the position information and structural brain data are used. The structural brain data may be indicative for the distribution and/or the structure of nerve fibers in the brain, particularly of the nerve fibers connecting the certain brain region with other brain regions. When tracking the nerve fibers by starting from the position of the certain brain region, only those nerve fibers which are indeed connected to the certain brain region may be tracked. The tracking of the nerve fibers starting from the position of the certain brain region is done, in particular, in order to determine the structural connection of the certain brain region to other brain regions via nerve fibers.

In order to track the nerve fibers, the position information and the information about the distribution/structure of nerve fibers in the structural brain data are, in particular, given with respect to the same coordinate system. The structural brain data may be computer data. For example, the distribution and/or the structure of nerve fibers can be extracted from the structural brain data, e.g. by using a computer program.

In at least one embodiment, the method for detecting nerve fibers in a brain of a patient comprises:
- receiving position information indicative for the position of a certain brain region of the brain,
- tracking nerve fibers starting from the position of the certain brain region in order to determine the nerve fibers connecting the certain brain region with other brain regions by using the position information and structural brain data, wherein
- the structural brain data are indicative for the distribution of nerve fibers in the brain.

The integrity of nerve fibers is biologically closely associated with psychological disturbances and relevant in personality psychology. Identifying a certain brain region, e.g. the locus coeruleus, and tracking nerve fibers from the position of the certain brain region allows to determine the structural connection of the certain brain region to other brain regions via the nerve fibers. For example, by comparing the structural connection to structural connections in other patients may help to identify psychological diseases.

The presented method is, inter alia, based on the idea to track nerve fibers by starting form a certain position, namely the position of a certain brain region. This allows identifying more precisely and more effectively those nerve fibers which are of interest, namely those which indeed connect the certain brain region to other brain regions.

According to at least one embodiment, the method is carried out *in vitro.* For example, the method is carried out without the necessity of the presence of the patient to which the position information and/or structural brain data belong. The method may be carried out hours or days after the structural brain data have been measured and/or after the position information have been determined.

According to at least one embodiment, the certain brain region is the locus coeruleus, LC for short. Particularly the integrity of LC's nerve fibers is biologically closely associated with psychological disturbances in neurodegenerative diseases related to LC (Alzheimer's, Parkinson's) and relevant in depression, post-traumatic stress disorder and anxiety.

According to at least one embodiment, the method is a computer-implemented method. Particularly, some or all steps of the method, especially the tracking of the nerve fibers, may be carried by a computer. Additionally or alternatively, tracking the nerve fibers is done by a fiber tracking algorithm. A commercial algorithm may be used for this purpose.

According to at least one embodiment, the position information is obtained by localizing the certain brain region using the structural brain data. In this case, the structural brain data are additionally indicative for the anatomy of the brain, particularly for the location of the certain brain region in the brain.

According to at least one embodiment, localizing the certain brain region is done manually by an operator. Alternatively, localizing the certain brain region may be done automatically with help of a computer. The position information obtained by localizing the certain brain region may then be entered and/or saved in the computer and used for tracking the nerve fibers.

According to at least one embodiment, the structural brain data comprise first data which are indicative for the position or location, respectively, of the certain brain region of the brain.

According to at least one embodiment, the structural brain data comprise second data which are indicative for the distribution of the nerve fibers in the brain.

The first data and the second data may be taken individually and/or independently of each other. For example, the first data and the second data may be taken in different measurement procedures. The first data and the second data may then be combined to the structural brain data. The structural brain data may also comprise third data which are indicative for the whole brain anatomy. In other words, the structural brain data may comprise at least two data sets, namely the first data and the second data and optionally also the third data.

According to at least one embodiment, localizing the certain brain region is done by using the first data. For example, an operator may manually investigate the first data in order to localize the certain brain region or a computer may be used to investigate the first data in order to localize the certain brain region.

According to at least one embodiment, tracking the nerve fibers is done by using the second data.

According to at least one embodiment, the first data and/or the second data and/or the third data are obtained by MRI (magnetic resonance imaging). For example, the first data, the second data and optionally the third data are obtained in different MRI sequences. Each of these MRI sequences may take several minutes, e.g. 15 minutes. The different MRI sequences may be performed one after the other and/or independently of each other. A measurement break, e.g. of several minutes, may lie between two MRI sequences.

According to at least one embodiment, the first data are obtained by a neuromelanin sensitive MRI sequence.

According to at least one embodiment, the certain brain region is localized based on the neuromelanin occurrence extractable from the first data. Particularly, the first data may be indicative for the neuromelanin occurrence in the brain as a function of the position in the brain. For example, the certain brain region is identified based on a threshold value of the neuromelanin occurrence. The neuromelanin occurrence may be extracted from an MRI signal or an MRI signal ratio. A region in the brain with a neuromelanin occurrence exceeding the threshold may be identified as the certain brain region.

According to at least one embodiment, the second data are obtained by a diffusion weighted MRI sequence. A diffusion weighted MRI sequence may also be referred to as diffusion tensor imaging, DTI for short.

According to at least one embodiment, the method comprises a step in which the number of tracked nerve fibers is determined. Particularly, the number of tracked nerve fibers connected to the certain brain region is determined. This step is, in particular, performed after tracking the nerve fibers.

According to at least one embodiment, the method comprises a step in which the number of tracked nerve fibers is normalized in order to make it comparable to numbers obtained from other patients. Normalizing may be done by z- transformation. For example, normalizing may be done by calculating z-scores, whereby the mean of tracked nerve fibers is subtracted from the individual numbers of tracked nerve fibers and the difference is divided by the standard deviation.

According to at least one embodiment, determining the number of tracked nerve fibers comprises applying a filter algorithm. This may be done in order to determine the number of biologically relevant or accurate nerve fibers. The filter algorithm may be Informed Filtering of Tractograms, like SIFT or SIFT2.

According to at least one embodiment, the method comprises a step of visualizing the brain and/or the certain brain region and/or the tracked nerve fibers. This may be done based on the structural brain data and/or tracking data. The tracking data may be data obtained from tracking the nerve fibers starting from the certain brain region and may be indicative for the distribution and/or structure of the tracked nerve fibers. Visualization may be done via displaying on a screen, e.g. a computer screen.

According to at least one embodiment, the method comprises a step in which the tracked nerve fibers or the tracking data, respectively, and functional brain data are combined in order to investigate the relation between the structural and the functional connection of the certain brain region to other brain regions. For example, the tracked nerve fibers/tracking data obtained from the structural brain data may be displayed together with information of the functional brain data, e.g. nerve fibers extracted from the functional brain data.

Additionally or alternatively, the first data are combined with the functional brain data or a re used for measuring the functional brain data. For example, the position information of the certain brain region is used for the measuring the functional brain data.

According to at least one embodiment, the functional brain data is obtained by resting-state functional MRI. In resting-state functional MRI, the blood stream in the brain is measured by a specific MRI sequence as a marker of brain activity, while the patient is not doing anything in the MRI ("resting state"). The sequence of resting-state functional MRI may be measured individually and/or independently of the other sequence(s) used for obtaining the structural brain data. The functional brain data may be used to compare the structural connectivity (the nerve fiber connection) of the certain brain region, e.g. the LC, with its "functional connectivity" (which reveals the simultaneous activity of brain regions with the certain brain region). The resting-state functional MRI may particularly be used to validate structural connectivity of the nerve fibers extracted from the structural brain data.

Next, the device, the computer program and the computer-readable data carrier are specified.

According to at least one embodiment, the device comprises at least one processor configured to perform the method for detecting nerve fibers specified herein.

According to at least one embodiment, the computer program comprises instructions which, when the program is executed by a computer, cause the computer to carry out the method for detecting nerve fibers specified herein.

According to at least one embodiment, the computer-readable data carrier has stored thereon the computer program.

Next, the method for diagnosing or prognosing a disease in or psychological characteristic of a patient is specified.

The involvement of the LC degeneration in psychiatric diseases is also well known from human and animal studies.

The invention relates to a method for diagnosing or prognosing a disease in or psychological characteristic of a patient comprising the step of:
(i) carrying out the method (for detecting nerve fibers in a brain of a patient) as specified above.

The term "disease", as used herein, refers to an abnormal condition that affects the body of an individual. A disease is often construed as a medical condition associated with specific symptoms and signs. A disease may be caused by factors originally from an external source or it may be caused by internal dysfunctions. In humans, the term "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behavior, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

The term "diagnosing a disease", as used herein, means determining whether an individual shows signs of or suffers from the disease. The term "prognosing a disease", as used herein, means determining whether there is a likelihood that the individual will show signs of or suffer from the disease.

The term "psychological characteristic or trait", as used herein, is a distinguishing feature or quality. In psychological terms, personality traits and behavioural characteristics define an individual. Psychologists often refer to personality traits or characteristics. The unique combination of personality traits makes individuals. Some examples of personality traits psychologists have identified and studied are the following: warmth, emotional stability, independence, dominance, impulsivity, sensitivity, imagination, introversion-extroversion, suspiciousness, or boldness. But, certain traits have become associated with psychological problems. In one preferred embodiment, the psychological characteristic is selected from the group consisting of arousal capacity, stress level, or any characteristic depending on the psychological function of the LC or its physiological noradrenaline related brain function.

The term "patient", as used herein, refers to any subject for whom it is desired to know whether she or he suffers from a disease or dysfunction. In particular, the term "patient", as used herein, refers to a subject suspected to be affected by a disease. The patient may be diagnosed to be affected by the disease, may be diagnosed to be not affected by the disease, i.e. healthy, or may be diagnosed to be not affected by the disease anymore (e.g. after therapeutic intervention). The term "patient", as used herein, further refers to any subject who is interested in his/her psychological characteristics. This subject does not necessarily have to be diagnosed with a disease or suffer from a disease.

According to at least one embodiment, the distribution of nerve fibers (NF) in the brain (B) is indicative for the presence of a disease in the patient (diagnosis), the likelihood of development of a disease in the patient (prognosis), or the psychological characteristic in the patient.

Thus, the determination of the distribution of nerve fibers (NF) in the brain (B) allows the diagnosis of a disease in the patient, the prognosis of a disease in the patient, or the psychological characteristic of the patient.

Since nerve fibers form an electrophysiological and/or neurotransmitter-based connectivity between brain regions, and since certain brain regions and certain nerve fiber (NF) connections have distinct functions, e.g. within cerebral signal processing or executive behavioral control, the distribution of nerve fibers (NF) directly influences brain functionality. For example, subjects with a high number of nerve fibers (NF) from brain region LC to brain region A compared to number of nerve fibers (NF) from region LC to brain region B may show disease or psychological characteristic related differences associated with the brain function of region A or function of LC-region A connectivity. Contrary, subjects with a high number or ratio of nerve fibers (NF) from brain region LC to brain region B compared to number of nerve fibers (NF) from region LC to brain region A may show disease or psychological characteristic related differences associated with the brain function of region B or function of LC-region B connectivity.

According to at least one preferred embodiment, the method further comprises the step of:
(ii) determining the number of nerve fibers (NF) connecting the certain brain region (BR) with other brain regions,
wherein the number of nerve fibers (NF) is indicative for the presence of a disease in the patient (diagnosis), the likelihood of development of a disease in the patient (prognosis), or the psychological characteristic of the patient.

Thus, the determination of the number of nerve fibers (NF) also allows the diagnosis of a disease in the patient, the prognosis of a disease in the patient, or the psychological characteristic of the patient.

It is, for example, preferred to define a specific threshold of the number of nerve fibers (NF). If the number of nerve fibers exceeds the specific threshold, the patient is diagnosed as suffering from the disease, prognosed as having a likelihood to develop the disease, or as having the specific psychological characteristic.

Alternatively, it is possible to determine the ratio of number of nerve fibers (NF) of two or more connections between brain regions and compare them with each other. In particular, it is alternatively possible to determine the ratio of number of nerve fibers (NF) of a certain brain region (BR) with other brain regions. Thus, in an alternative, it is the ratio of number of nerve fibers (NF) who is indicative for the presence of a disease in the patient (diagnosis), the likelihood of development of a disease in the patient (prognosis), or the psychological characteristic of the patient.

According to at least one embodiment, the psychological characteristic is selected from the group consisting of arousal capacity, stress level, or any characteristic depending on the psychological function of the LC or its physiological noradrenaline related brain function.

The physiological noradrenaline related brain function of the LC is the result of its control over most of the release of the neurotransmitter noradrenaline into the whole brain and peripheral body, and hereby its predominant control over the sympathetic nervous system, which includes its modulating influence on organs like the heart, kidney, vessel contractility or immune system. Noradrenaline influences functionality of a variety of cell types and e.g. influences signal transmission, signal storage or cell proliferation. Hereby, noradrenaline binding on adrenal cell receptors plays a major role, and its consequent action may be summarized as a major drive for human physical and psychological performance.

The psychological function of the LC refers to the aforementioned physiological mechanisms, and e.g. includes its influence on vigilance, speed of reaction, memory control, fear, pain, stress, and the coherent physiological reactions like heart rate, blood pressure, pupil reactions, or body temperature homeostasis.

According to at least one embodiment, the certain brain region (BR) is the locus coeruleus (LC). The LC as the primary source of noradrenaline (NA) in the human brain has been functionally and anatomically studied for decades based on anatomical surveys and electrophysiological measures. Its central importance for key capabilities of the brain like cognitive control, arousal and attention, memory, and emotions have widely been confirmed based on task studies in both animal and human experiments. Particularly the integrity of LC's nerve fibers is biologically closely associated with psychological disturbances in neurodegenerative diseases related to LC (Alzheimer's, Parkinson's) and relevant in depression, post-traumatic stress disorder and anxiety. The functionality of the LC and consequently the differential disturbances in the associated diseases lie in the spillover of NA throughout its long-reaching nerve fibers.

Thus, according to at least one preferred embodiment, the disease is a LC-related disease. LC-related disease refers to all disturbances in physiological noradrenaline related brain function or psychological function of the LC.

According to at least one preferred embodiment, the LC-related disease is a neurodegenerative disease, a psychiatric disease, or a disease associated with the psychological function of the LC.

From post-mortem anatomical sections it is known that in Alzheimer disease the locus coeruleus (LC) and its nerve fibers are the first structures that degenerate in the brain, which makes it interesting as an early marker for the disease and its progression. The LC and its nerve fibers degenerate years before the patients suffer from any symptoms. Furthermore, the LC degeneration correlates with disease severity.

Thus, in one more preferred embodiment,
the neurodegenerative disease is selected from the group consisting of Alzheimer's Disease (AD), Parkinson's Disease (PD), or
the psychiatric disease is selected from the group consisting of depression, anxiety disorders, posttraumatic stress disorder (PTSD), and attention-deficit/hyperactivity disorder (ADHD).

According to at least one embodiment, the method is carried out *in vitro.* For example, the method is carried out without the necessity of the presence of the patient to which the position information and/or structural brain data belong. The method may be carried out hours or days after the structural brain data have been measured and/or after the position information have been determined.

Next, the method of determining the course of a disease in a patient or psychological characteristic of a patient is specified.

The invention relates to a method of determining the course of a disease in or psychological characteristic of a patient comprising the step of:
(i) carrying out the method (for detecting nerve fibers in a brain of a patient) as specified above.

The term "determining the course of a disease or psychological characteristic", as used herein, means determining the development of the disease or psychological characteristic over time, e.g. whether the disease in or psychological characteristic of a patient worsens, does not worsen/is stable, or improves over time.

According to at least one embodiment, the method comprises the steps of:
(i) carrying out the method (for detecting nerve fibers in a brain of a patient) as specified above at a first point in time, and
(ii) carrying out the method (for detecting nerve fibers in a brain of a patient) as specified above at least at a second point in time,
(iii) comparing the distribution of nerve fibers (NF) in the brain (B) determined at the different time points, wherein the change of the distribution of nerve fibers (NF) in the brain (B) is indicative for the course of the disease in or psychological characteristic of the patient.

Thus, the determination of the distribution of nerve fibers (NF) in the brain (B) allows to monitor or track the disease in the patient or psychological characteristic of the patient. In particular, the determination of a disease or psychological characteristic progression depends on the change of the distribution of nerve fibers (NF) in the brain (B) over time, a disease or psychological characteristic stabilization depends on the change of the distribution of nerve fibers (NF) in the brain (B) over time, or the disease or psychological characteristic improvement depends on the change of the distribution of nerve fibers (NF) in the brain (B) over time.

According to at least one preferred embodiment, the method comprises the steps of:
(i) at a first point in time
   (ia) carrying out the method (for detecting nerve fibers in a brain of a patient) as specified above and
   (ib) determining the number of nerve fibers (NF) connecting the certain brain region (BR) with other brain regions, and
(ii) at least at a second point in time
   (iia) carrying out the method (for detecting nerve fibers in a brain of a patient) as specified above, and
   (iib) determining the number of nerve fibers (NF) connecting the certain brain region (BR) with other brain regions, and
(iii) comparing the numbers of nerve fibers (NF) determined at the different time points, wherein the change of the number of nerve fibers (NF) over time is indicative for the course of the disease in or psychological characteristic of the patient.

Thus, the determination of the number of nerve fibers (NF) also allows to monitor or track the disease in the patient or psychological characteristic of the patient. In particular, the determination of a disease or psychological characteristic progression depends on the change of the number of nerve fibers (NF) over time, a disease or psychological characteristic stabilization depends on the change of the number of nerve fibers (NF) over time, or the disease or psychological characteristic improvement depends on the change of the number of nerve fibers (NF) over time.

Alternatively, it is possible to determine the ratio of number of nerve fibers (NF) of two or more connections between brain regions and compare them with each other. In particular, it is alternatively possible to determine the ratio of number of nerve fibers (NF) of a certain brain region (BR) with other brain regions at the different time points. Thus, in an alternative, it is the change of the ratio of number of nerve fibers (NF) determined at the different time points which is indicative for the course of the disease in or psychological characteristic of the patient.

The time period between the first point in time and the later point(s) in time preferably amounts to at least 1 day, at least 2 days, at least 3 days, at least 4 days, at least 5 days, at least 6 days, at least 7 days (1 week), at least 2 weeks, at least 3 weeks, at least 4 weeks, at least 1 month, at least 2 months, at least 3 months, at least 4 months, at least 5 months, at least 6 months, at least 7 months, at least 8 months, at least 9 months, at least 10 months, at least 11 months, at least 12 months (1 year), at least 24 months (2 years), at least 3 years, at least 4 years, at least 5 years, at least 6 years, at least 7 years, at least 8 years, at least 9 years, or at least 10 years. For example, the patient may be routinely checked, e.g. once or twice a year. The patient may be (re)tested at 2, 3, 4, 5, 6 7, 8, 9, or 10 time points (first point in time and further point(s) in time).

In addition to the determination of the course of the disease in or psychological characteristic of a patient, the treatment of this disease or psychological characteristic can be monitored. It is namely preferred that the patient receives, has received, or had received a treatment, in particular therapeutic treatment.

The term "treatment", in particular "therapeutic treatment", as used herein, refers to any therapy which improves the health status and/or prolongs (increases) the lifespan of a patient. Said therapy may eliminate the disease in a patient, arrest or slow the development of a disease in a patient, inhibit or slow the development of a disease in a patient, decrease the frequency or severity of symptoms in a patient, and/or decrease the recurrence in a patient who currently has or who previously has had a disease.

The treatment may be selected from the group consisting of the administration of a drug, exercise training, mental training, and physical rehabilitation.

The patient may receive a treatment during the complete determination/monitoring process (e.g. the administration of a drug) or may receive a treatment before, at, or after a first point in time (e.g. the administration of a drug) and may be retested at a later point in time. In particular, said first point in time may be before the initiation of a treatment and said later point in time may be during the treatment and/or after the treatment. If the treatment encompasses the administration of a drug and the patient responds to said treatment, the drug administration may be continued, the dose of the drug may be reduced, or the drug administration may be stopped. If the treatment encompasses the administration of a drug and the patient does not respond to said treatment, the dose of the drug may be increased, the drug may be changed, or the therapy mode may be changed, e.g. from drug administration to exercise training, mental training or physical rehabilitation.

According to at least one embodiment, the psychological characteristic is selected from the group consisting of arousal capacity, stress level, or any characteristic depending on the psychological function of the LC or its physiological noradrenaline related brain function.

According to at least one embodiment, the certain brain region (BR) is the locus coeruleus, LC for short.

Thus, according to at least one preferred embodiment, the disease is a LC-related disease.

According to at least one preferred embodiment, the LC-related disease is a neurodegenerative disease, a psychiatric disease, or a disease associated with the psychological function of the LC.

In one more preferred embodiment,
the neurodegenerative disease is selected from the group consisting of Alzheimer's Disease (AD), Parkinson's Disease (PD), or
the psychiatric disease is selected from the group consisting of depression, anxiety disorders, posttraumatic stress disorder (PTSD), and attention-deficit/hyperactivity disorder (ADHD).

According to at least one embodiment, the method is carried out *in vitro.*

Hereinafter, the method for detecting nerve fibers described herein will be explained in more detail with reference to drawings on the basis of exemplary embodiments. Same reference signs indicate same elements in the individual figures. However, the size ratios involved are not necessarily to scale, individual elements may rather be illustrated with exaggerated size for better understanding.
**Figure 1** shows images of brains of three different patients,
**Figure 2** shows images of the brain of a patient in different views and extracted from different kind of data.
**Figure 3** shows a flowchart of an exemplary embodiment of the method for detecting nerve fibers in a brain of a patient,
**Figure 4** shows images of the brain of a patient taken with a neuromelanin sensitive MRI sequence.

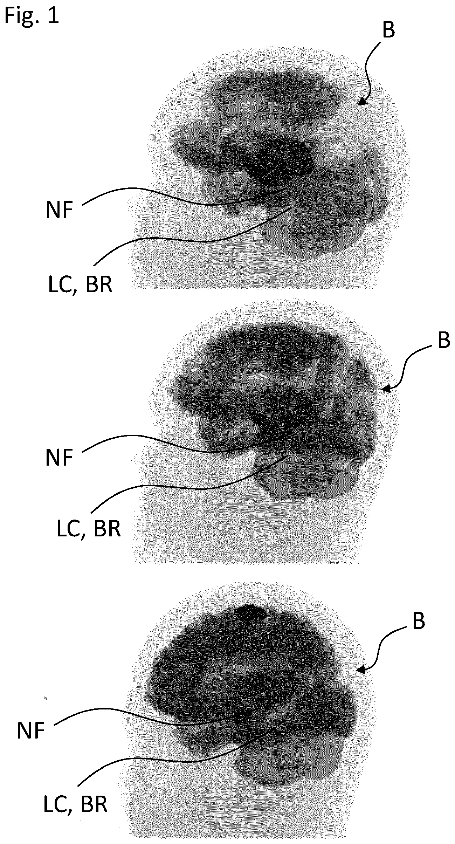

**Figure 1** shows images of brains B of three different patients. The brains B were visualized by using structural brain data which were taken by MRI. The structural brain data of each patient comprise three different data sets, namely first data, second data and third data. The three data sets were each obtained by an individual MRI sequence. The first data was obtained by a neuromelanin sensitive MRI sequence, the second data was obtained by a diffusion weighted MRI sequence and the third data was obtained by a structural MRI sequence for receiving the whole brain anatomy. Information of these three data are shown in an overlaid fashion in figure 1.

The structural MRI sequence and the neuromelanin sensitive sequence were performed using a Siemens MAGNETOM Prisma 3T MRI scanner with syngo MR E11 software and a 64-channel head coil with the following parameters for the anatomical scans: 3D-MPRAGE sequence, echo time (TE) = 2.82 ms, repetition time (TR) = 2,500 ms, inversion time (T1) = 1,100 ms, flip angle = 7deg, bandwidth = 140 Hz/pixel, acquisition volume = 256 x 256 x 192 mm³, isometric voxel size = 1.0 mm³, scan duration = 5 min 18 s.

Neuromelanin acts as an endogenous MR contrast agent: it shortens the longitudinal relaxation time T1 and the LC appears hyperintense in high-resolution T1-weighted MRI. A T1-weighted TSE sequence was applied for taking the neuromelanin sensitive sequence with the following imaging parameters: 14 axial slices, acquisition volume = 192 x 192 x 42 mm³, slice thickness = 2.50 mm, inter-slice gap 0.5 mm, leading to an effective slice thickness of 3 mm, TR = 634.0 ms, TE = 10.0 ms, bandwidth = 165 Hz/Pixel, flip angle = 80deg, scan duration = 10 min 50s.

The diffusion weighted sequence was acquired with a monopolar single-shot spin echo EPI sequence: TE = 74 ms; TR = 4970 ms; flip angle a = 90°; parallel GRAPPA acceleration factor = 2, matrix: 130 × 130; FOV = 208 × 208 mm²; spatial resolution = 1.6 × 1.6 × 1.6 mm³; multiband acceleration factor = 2; phase-encoding direction: anterior >> posterior; 228 isotropically distributed diffusion sensitization directions (38 at b = 1,000 s/mm², 76 at b = 2,000 s/mm², and 114 at b = 3,000 s/mm²) and 14 b = 0 s/mm2 images (interspersed throughout the acquisition) were collected. To generate appropriate field maps to correct for susceptibility-induced distortions, nine b = 0 s/mm² images with reversed phase encoding (posterior >> anterior) were also acquired. The total scan duration was 22 min 31 s.

The location of a certain brain region BR, namely the locus coeruleus LC, was done by using the first data. This step may be done manually by an operator or automatically with help of a computer. The nerve fibers NF were tracked from the position of the locus coeruleus LC using the second data obtained by the diffusion weighted MRI sequence. The tracked nerve fibers NF were filtered using informed filtering of tractograms (SIFT2) and were normalized using z-transformation. The tracked nerve fibers NF were then visualized and overlaid to the third data representing the whole brain anatomy.

As can be seen in **Figure 1****,** the different patients have different numbers of nerve fibers connecting the locus coeruleus LC with other brain regions. The visibility, or more specifically the opacity of the presented brain regions is proportional to the number of nerve fibers, which connect the regions with the LC, to additionally provide an 'at the first sight' visual assessment beyond the illustration of the nerve fibers. This information can be used to diagnose or prognose a disease in or psychological characteristics of the patient and/or to determine the course of a disease in or psychological characteristics of the patient.

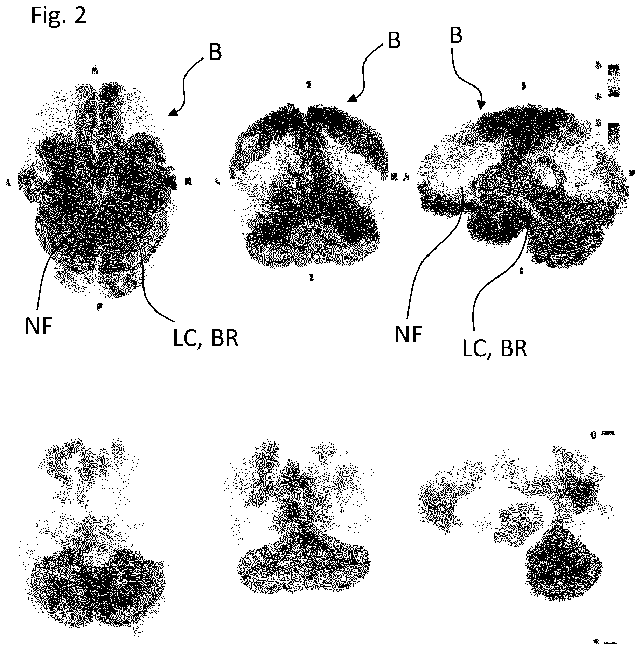

**Figure 2** shows, in the upper row, the distribution of nerve fibers NF tracked from the position of the locus coeruleus LC by using structural brain data. The lower row shows results obtained from functional brain data illustrating the functional connection of the locus coeruleus LC to other brain regions. The functional brain data were obtained by a resting-state MRI sequence.
The comparison between upper and lower row shows the concordance of number of nerve fiber connections (NF, upper row) and strength of functional connections (lower row) from the LC to subcortical brain structures.

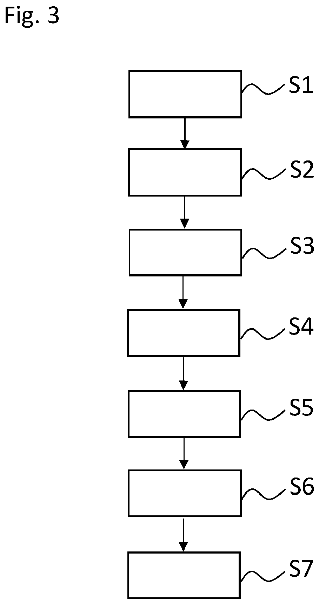

**Figure 3** shows an exemplary embodiment of the method for detecting nerve fibers NF in the brain B of a patient. In a first step S1, a certain brain region BR, e.g. the locus coeruleus LC, is localized. For this purpose, the above-mentioned first data may be used. The position information of the certain brain region BR is then received in a step S2. For example, the position information is manually entered into a computer and/or is received by a processor. In a step S3, nerve fibers NF are tracked starting from the position of the certain brain region BR in order to determine the nerve fibers NF connecting the certain brain region BR with other brain regions by using the position information and structural brain data, particularly the above-mentioned second data. The structural brain data or the second data, respectively, are indicative for the distribution of nerve fibers in the brain. In a step S4, the number of nerve fibers NF, particularly of nerve fibers NF connected to the certain brain region BR, is determined. For this purpose, SIFT2 may be used. Then, in a step S5, the number of determined nerve fibers NF is normalized, e.g. by using z-transformation. This may be done in order to make the determined number comparable to numbers obtained from other patients. In a step S6, the structural information obtained in the steps S1 to S5 may be compared to functional brain data, e.g. in order to validate the results of the steps S1 to S5. This may be done visualizing the tracked nerve fibers and information of the functional brain data on a screen.
In a step S7, the structural and/or the functional data prepared in steps S1 to S6 may be visually or computationally compared to a normative sample, individual healthy controls, to a previous data assessment of the patient, analyzed within the actual data of the patient, or to/within a combination of those options.

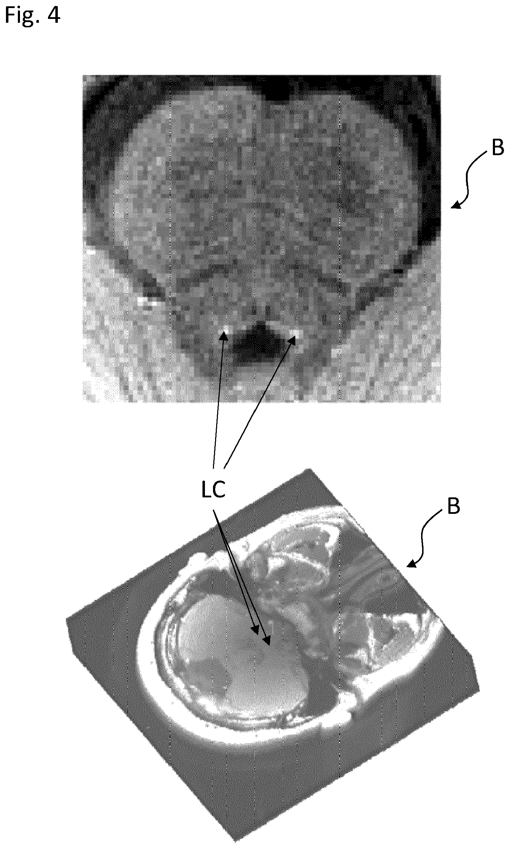

**Figure 4** shows a visualization of the first data taken with the neuromelanin sensitive MRI sequence and being indicative for the position of the locus coeruleus LC. The upper image of figure 4 shows a section of the brain B obtained from the first data and used for identifying the locus coeruleus LC and the lower image shows a 3D representation of the brain B obtained from the first data. The locus coeruleus LC is localized based on the neuromelanin occurrence in the visualized first data. The determined position of the locus coeruleus LC is indicated in the upper and lower image.

In the following, a set of embodiments is disclosed. The embodiments are numbered to facilitate referencing the features of one embodiment in other embodiments. The embodiments form part of the disclosure of the present application and could be made subject to independent and/or dependent claims irrespective of what currently is claimed in the application and also independent of the references in brackets. We note, however, that the scope of protection is defined by the appended claims, where the following embodiments do not constitute claims. The embodiments are:
1. A method for detecting nerve fibers (NF) in a brain (B) of a patient, comprising
   - receiving position information indicative for the position of a certain brain region (BR) of the brain (B),
   - tracking nerve fibers (NF) starting from the position of the certain brain region (BR) in order to determine the nerve fibers (NF) connecting the certain brain region (BR) with other brain regions by using the position information and structural brain data, wherein
   - the structural brain data are indicative for the distribution of nerve fibers (NF) in the brain (B).
2. The method according to embodiment 1, wherein the method is carried out *in vitro.*
3. The method according to embodiment 1 or 2, wherein the certain brain region (BR) is the locus coeruleus (LC).
4. The method according to any one of the preceding embodiments, wherein
   - the method is a computer-implemented method and/or tracking the nerve fibers (NF) is done by a fiber tracking algorithm.
5. The method according to any one of the preceding embodiments, wherein
   - the position information is obtained by localizing the certain brain region (BR) using the structural brain data, wherein the structural brain data are additionally indicative for the anatomy of the brain (B).
6. The method according to embodiment 5, wherein
   - localizing the certain brain region (BR) is done manually by an operator or automatically with help of a computer.
7. The method according to embodiment 5 or 6, wherein
   - the structural brain data comprise first data which are indicative for the position of the certain brain region in the brain (B) and second data which are indicative for the distribution of nerve fibers (NF) in the brain (B),
   - localizing the certain brain region (BR) is done by using the first data,
   - tracking the nerve fibers (NF) is done by using the second data,
   - the first data and the second data are obtained by MRI.
8. The method according to embodiment 7, wherein
   - the first data are obtained by a neuromelanin sensitive MRI sequence,
   - the certain brain region (BR) is localized based on the neuromelanin occurrence extractable from the first data,
   - the second data are obtained by a diffusion weighted MRI sequence.
9. The method according to any one of the preceding embodiments, wherein
   - the number of tracked nerve fibers (NF) is determined,
   - the number is normalized in order to make it comparable to numbers obtained from other patients.
10. The method according to embodiment 9, wherein
   - determining the number of tracked nerve fibers (NF) comprises applying Informed Filtering of Tractograms, SIFT2.
11. The method according to any one of the preceding embodiments, further comprising
   - visualizing the brain (B), the certain brain region (BR) and/or the tracked nerve fibers (NF).
12. The method according to embodiment 11, wherein
   - the brain (B), the localized brain region (BR) and/or the tracked nerve fibers (NF) are displayed on a screen.
13. The method according to any one of the preceding embodiments, further comprising
   - combining the tracked nerve fibers (NF) and functional brain data in order to investigate the relation between the structural and the functional connection of the certain brain region (BR) to other brain regions.
14. The method according to embodiment 13 wherein
   - the functional brain data is obtained by resting-state functional MRI.
15. A device comprising at least one processor configured to perform the method of any one of the preceding embodiments.
16. A computer program comprising instructions which, when the program is executed by a computer, cause the computer to carry out the method of any one of the embodiments 1 to 14.
17. A computer-readable data carrier having stored thereon the computer program of the preceding embodiment.
18. A method for diagnosing or prognosing a disease in or psychological characteristic of a patient comprising the step of:
   (i) carrying out the method of any one of embodiments 1 to 14.
19. The method of embodiment 18, wherein the distribution of nerve fibers (NF) in the brain (B) is indicative for the presence of a disease in the patient (diagnosis), the likelihood of development of a disease in the patient (prognosis), or the psychological characteristic of the patient.
20. The method of embodiments 18 or 19, wherein the method further comprises the step of:
   (ii) determining the number of nerve fibers (NF) connecting the certain brain region (BR) with other brain regions,
   wherein the number of nerve fibers (NF) is indicative for the presence of a disease in the patient (diagnosis), the likelihood of development of a disease in the patient (prognosis), or the psychological characteristic of the patient.
21. The method of any one of embodiments 18 to 20, wherein the psychological characteristic is selected from the group consisting of arousal capacity, stress level, or any characteristic depending on the psychological function of the LC or its physiological noradrenaline related brain function.
22. The method of any one of embodiments 18 to 21, wherein the certain brain region (BR) is the locus coeruleus (LC).
23. The method of any one of embodiments 18 to 22, wherein the disease is a LC-related disease.
24. The method of embodiment 23, wherein the LC-related disease is a neurodegenerative disease, a psychiatric disease, or a disease associated with the psychological function of the LC.
25. The method of embodiment 24, wherein
   the neurodegenerative disease is selected from the group consisting of Alzheimer's Disease (AD), Parkinson's Disease (PD), or
   the psychiatric disease is selected from the group consisting of depression, anxiety disorders, posttraumatic stress disorder (PTSD), and attention-deficit/hyperactivity disorder (ADHD).
26. The method of any one of embodiments 18 to 25, wherein the method is carried out *in vitro.*
27. A method of determining the course of a disease in or psychological characteristic of a patient comprising the step of:
   (i) carrying out the method of any one of embodiments 1 to 14.
28. The method of embodiment 27, wherein the method comprises the steps of:
   (i) carrying out the method of any one of embodiments 1 to 14 at a first point in time, and
   (ii) carrying out the method of any one of embodiments 1 to 14 at least at a second point in time,
   (iii) comparing the distribution of nerve fibers (NF) in the brain (B) determined at the different time points, wherein the change of the distribution of nerve fibers (NF) in the brain (B) is indicative for the course of the disease in or psychological characteristic of the patient.
29. The method of embodiments 27 or 28, wherein the method comprises the steps of:
   (i) at a first point in time
      (ia) carrying out the method of any one of embodiments 1 to 14 and
      (ib) determining the number of nerve fibers (NF) connecting the certain brain region (BR) with other brain regions, and
   (ii) at least at a second point in time
      (iia) carrying out the method of any one of embodiments 1 to 14 and
      (iib) determining the number of nerve fibers (NF) connecting the certain brain region (BR) with other brain regions, and
   (iii) comparing the numbers of nerve fibers (NF) determined at the different time points, wherein the change of the number of nerve fibers (NF) over time is indicative for the course of the disease in or psychological characteristic of the patient.
30. The method of any one of embodiments 27 to 29, wherein the psychological characteristic is selected from the group consisting of arousal capacity, stress level, or any characteristic depending on the psychological function of the LC or its physiological noradrenaline related brain function.
31. The method of any one of embodiments 27 or 30, wherein the certain brain region (BR) is the locus coeruleus (LC).
32. The method of any one of embodiments 27 to 31, wherein the disease is a LC-related disease.
33. The method of embodiment 32, wherein the LC-related disease is a neurodegenerative disease, psychiatric disease, or a disease associated with the psychological function of the LC or its physiological noradrenaline related brain function.
34. The method of embodiment 33, wherein
   the neurodegenerative disease is selected from the group consisting of Alzheimer's Disease (AD), Parkinson's Disease (PD, or
   the psychiatric diseases is selected from the group consisting of depression, anxiety disorders, posttraumatic stress disorder (PTSD), and attention-deficit/hyperactivity disorder (ADHD).
35. The method of any one of embodiments 27 to 34, wherein the method is carried out *in vitro.*

The invention described herein is not limited by the description in conjunction with the exemplary embodiments. Rather, the invention comprises any new feature as well as any combination of features, particularly including any combination of features in the patent claims, even if said feature or said combination per se is not explicitly stated in the patent claims or exemplary embodiments.

### REFERENCE NUMBER LIST

- B: brain
- BR: certain brain region
- LC: locus coeruleus
- NF: nerve fibers
- S1 ... S7: method steps

## Claims

1. A method for detecting nerve fibers (NF) in a brain (B) of a patient, comprising
- receiving position information indicative for the position of a certain brain region (BR) of the brain (B),
- tracking nerve fibers (NF) starting from the position of the certain brain region (BR) in order to determine the nerve fibers (NF) connecting the certain brain region (BR) with other brain regions by using the position information and structural brain data, wherein
- the structural brain data are indicative for the distribution of nerve fibers (NF) in the brain (B).

2. The method according to claim 1, wherein the certain brain region (BR) is the locus coeruleus (LC).

3. The method according to any one of the preceding claims, wherein
- the position information is obtained by localizing the certain brain region (BR) using the structural brain data, wherein the structural brain data are additionally indicative for the anatomy of the brain (B),
- localizing the certain brain region (BR) is done manually by an operator or automatically with help of a computer.

4. The method according to claim 3, wherein
- the structural brain data comprise first data which are indicative for the position of the certain brain region in the brain (B) and second data which are indicative for the distribution of nerve fibers (NF) in the brain (B),
- localizing the certain brain region (BR) is done by using the first data,
- tracking the nerve fibers (NF) is done by using the second data,
- the first data and the second data are obtained by MRI.

5. The method according to claim 4, wherein
- the first data are obtained by a neuromelanin sensitive MRI sequence,
- the certain brain region (BR) is localized based on the neuromelanin occurrence extractable from the first data,
- the second data are obtained by a diffusion weighted MRI sequence.

6. The method according to any one of the preceding claims, wherein
- the number of tracked nerve fibers (NF) is determined,
- the number is normalized in order to make it comparable to numbers obtained from other patients,
- determining the number of tracked nerve fibers (NF) comprises applying Informed Filtering of Tractograms, SIFT2.

7. The method according to any one of the preceding claims, further comprising
- combining the tracked nerve fibers (NF) and functional brain data in order to investigate the relation between the structural and the functional connection of the certain brain region (BR) to other brain regions,
- the functional brain data is obtained by resting-state functional MRI.

8. A device comprising at least one processor configured to perform the method of any one of the preceding claims.

9. A computer program comprising instructions which, when the program is executed by a computer, cause the computer to carry out the method of any one of the claims 1 to 7.

10. A computer-readable data carrier having stored thereon the computer program of the preceding claim.

11. A method for diagnosing or prognosing a disease in or psychological characteristic of a patient comprising the step of:
(i) carrying out the method of any one of claims 1 to 7.

12. The method of claim 11, wherein the distribution of nerve fibers (NF) in the brain (B) is indicative for the presence of a disease in the patient (diagnosis), the likelihood of development of a disease in the patient (prognosis), or the psychological characteristic of the patient, and wherein the method preferably further comprises the step of:
(ii) determining the number of nerve fibers (NF) connecting the certain brain region (BR) with other brain regions,
wherein the number of nerve fibers (NF) is indicative for the presence of a disease in the patient (diagnosis), the likelihood of development of a disease in the patient (prognosis), or the psychological characteristic of the patient.

13. A method of determining the course of a disease in or psychological characteristic of a patient comprising the step of:
(i) carrying out the method of any one of claims 1 to 7.

14. The method of claim 13, wherein the method comprises the steps of:
(i) carrying out the method of any one of claims 1 to 7 at a first point in time, and
(ii) carrying out the method of any one of claims 1 to 7 at least at a second point in time,
(iii) comparing the distribution of nerve fibers (NF) in the brain (B) determined at the different time points, wherein the change of the distribution of nerve fibers (NF) in the brain (B) is indicative for the course of the disease in or psychological characteristic of the patient.

15. The method of claims 13 or 14, wherein the method comprises the steps of:
(i) at a first point in time
(ia) carrying out the method of any one of claims 1 to 7 and
(ib) determining the number of nerve fibers (NF) connecting the certain brain region (BR) with other brain regions, and
(ii) at least at a second point in time
(iia) carrying out the method of any one of claims 1 to 7 and
(iib) determining the number of nerve fibers (NF) connecting the certain brain region (BR) with other brain regions, and
(iii) comparing the numbers of nerve fibers (NF) determined at the different time points, wherein the change of the number of nerve fibers (NF) over time is indicative for the course of the disease in or psychological characteristic of the patient.
